# EUROPEAN PATENT SPECIFICATION

(11) **EP 4 221 706 B1**
(45) Date of publication and mention of the grant of the patent: **15.04.2026**
(21) Application number: 21783009.0
(22) Date of filing: 30.09.2021
(51) Int. Cl.: A61K 31/4545, A61K 31/496, A61K 31/501, A61K 31/506, A61K 31/5377, A61K 31/675, A61P 31/14, A61P 31/16

(54) **TREATMENT FOR VIRAL INFECTION**
BEHANDLUNG VON VIRUSINFEKTIONEN
TRAITEMENT D'INFECTION VIRALE

(30) Priority: 01.10.2020 GB 202015584
(43) Date of publication of application: 09.08.2023
(73) Proprietor: Oslo Universitetssykehus HF, 0424 Oslo (NO); Universitetet I Oslo, 0316 Oslo (NO)
(72) Inventor: FARHAN, Hesso, 6020 Innsbruck (AT); MUNTHE, Ludvig, 1472 Fjellhamar (NO); GRØDELAND, Gunnveig, 1386 Asker (NO); ROSENDAL, Ken, 0316 Oslo (NO)
(74) Representative: Dehns
(86) International application number: PCT/EP2021/077037
(87) International publication number: WO 2022/069684

(56) References cited:
- US-A1- 2019 167 710
- YI DONGRONG ET AL: "Identification of a Broad-Spectrum Viral Inhibitor Targeting a Novel Allosteric Site in the RNA-Dependent RNA Polymerases of Dengue Virus and Norovirus", FRONTIERS IN MICROBIOLOGY, vol. 11, 25 June 2020 (2020-06-25), XP055852253, DOI: 10.3389/fmicb.2020.01440
- NAVEEN KUMAR ET AL: "Receptor Tyrosine Kinase Inhibitors That Block Replication of Influenza A and Other Viruses", ANTIMICROBIAL AGENTS AND CHEMOTHERAPY, vol. 55, no. 12, 11 December 2011 (2011-12-11), US, pages 5553 - 5559, XP055559163, ISSN: 0066-4804, DOI: 10.1128/AAC.00725-11

## Description

### Field

The present disclosure relates to a leukocyte tyrosine kinase (LTK) inhibitor for use in the treatment of a viral infection in a subject, wherein the viral infection is caused by an RNA virus. The LTK inhibitor may be an acyclic ALK (anaplastic lymphoma kinase) inhibitor such as ceritinib, brigatinib, ensartinib, alectinib, crizotinib, entrectinib, belizatinib, CEP-28122 or CEP-37440. In particular, the disclosure relates to the treatment of influenza in human subjects.

### Background

Viral diseases pose a substantial and increasing disease burden on the human population with high morbidity and mortality. Globally, seasonal influenza (or flu) causes hundreds of thousands of deaths every year (despite the existence of vaccines) while many other potentially lethal viruses, for which no vaccines or specific treatments exist, are now endemic across much of the world, such as Dengue virus and West Nile virus.

In addition to the general viral disease burden, certain viruses have pandemic potential. This has been demonstrated to devastating effect by the 2020 COVID-19 pandemic (caused by the coronavirus SARS-CoV-2), while recent years have also witnessed outbreaks of Ebola virus in West Africa, Middle Eastern Respiratory Syndrome (MERS) and Severe Acute Respiratory Syndrome (SARS). Both MERS and SARS are also caused by coronaviruses (MERS-CoV and SARS-CoV-1, respectively). The enduring threat of an influenza pandemic is also a public health concern, particularly in respect of Highly Pathogenic Avian Influenza (HPAI), primarily of the subtypes H5N1 and H7N9 which have emerged in East Asia in recent decades.

Newly emerging coronaviruses (such as those mentioned above) and HPAI viruses have particular pandemic potential and can cause fatal acute lung injury. The mechanisms, cells and inflammatory mediators are only partly characterised for SARS-CoV-2, but it is known to cause diffuse alveolar damage (DAD) and cytokine storm (Guo et al., Military Medical Research 7: 11, 2020). This is similar to SARS-CoV-1, where mortality was generally a result of acute lung injuries in association with cytokine storms (IFN-γ, IL-1, IL-6, IL-12, CCL2, CXCL10, CXCL9, and IL-8) and low levels of IL-10 (Zhang et al., Infect Immun 72: 4410-4415, 2004). Alveoli were described to contain massive mixed inflammatory infiltrates (activated Tₕ1 and cytotoxic T cells, macrophages and NK-cells), epithelial necrosis and erosions, vascular leakage, fibrin and hyaline deposition, squamous metaplasia, and multinucleated giant cells. HPAI and MERS have similarly been associated with cytokine storms and acute lung injury.

While the ultimate aim in any pandemic situation is a vaccine that can be used to eliminate the spread of infection, vaccine development is a lengthy process. The development of treatments for viral infections is therefore essential in order to reduce mortality. To date, however, research into treatments for viral infections, such as anti-virals, has been limited and severely underfunded (with the exception of treatments for HIV).

Currently, treatments for respiratory viruses with pandemic potential (e.g. coronaviruses and influenza) are limited: corticosteroids such as dexamethasone are the only drugs to have been shown to significantly reduce mortality in COVID-19 patients (The RECOVERY Collaborative Group, N Engl J Med NEJMoa2021436, 2020), presumably by dampening the aberrant immune response that causes cytokine storms. However, corticosteroid therapy has been found to be ineffective in MERS treatment. The viral RNA-dependent RNA polymerase inhibitor remdesivir has activity against coronavirus polymerases and has been shown to reduce recovery time in COVID-19 patients (Beigel et al., N Engl J Med NEJMoa2007764, 2020), though trial results in respect of its impact on mortality are so far inconclusive.

A handful of influenza drugs have historically been globally available: the adamantane derivatives rimantadine and amantadine and the neuraminidase inhibitors (NAls) oseltamivir and zanamivir. However, from 2004 the adamantane derivatives were no longer recommended due to the emergence of resistance in most circulating influenza viruses. Resistance to NAls has also been reported, e.g. 90 % of circulating strains exhibited resistance to oseltamivir during the 2007-2009 influenza seasons (Hurt et al., N Engl J Med 365: 2541-2542, 2011).

More recently, two further NAls (laninamivir and peramivir) have been introduced in Japan, China, South Korea, and the USA. These have been licensed for influenza prevention and treatment. In 2018 the RNA-dependent RNA polymerase inhibitors baloxavir marboxil (BAM), and favipiravir reached the market (the first in Japan and the USA, and the latter in Japan), these drugs inhibit viral replication. However, use of these drugs was associated with a rapid emergence of resistant strains and mutated viruses could be identified in 2.2-9.7% of cases (Hayden et al., N Engl J Med 379: 913-923, 2018). Kumar et al., Antimicrobial Agents and Chemotherapy, vol. 55, no. 12, 2011, p. 5553-5559, discloses receptor tyrosine kinase inhibitors (RTKi) for use in the treatment of influenza infection.

The development of antiviral drugs for use in pandemic scenarios is challenging, since many drugs are effective against only a small subset of viruses and so may not be effective against an emerging virus. Moreover, many viruses, particularly RNA viruses such as influenza, have a high rate of mutation meaning that drug-resistant strains can rapidly emerge. To address both these challenges, there is a need for new drugs for treatment of viral infections that target host mechanisms that are hijacked by viruses, rather than viruses themselves. Wide ranges of virus types hijack the same host mechanisms during infection, meaning drugs which target these mechanisms can be expected to have a broad spectrum of activity, including against new or emerging viruses. Moreover, targeting a host pathway will negate development of viral escape mutations and serve as a durable and efficient druggable target in severe infections.

In COVID-19, fatal lung injury occurs only in a small subset of the population and, without being bound by theory, one hypothesis is that lung injury caused by novel coronavirus infection is caused by insufficient immunity and unchecked secretion of the virus that over-activates macrophages, resulting in cytokine storms (Liu et al., JCI Insight 4(4): e123158, 2019). If so, reducing the viral load is a key step in preventing mortality in COVID-19 and other such infections.

The receptor tyrosine kinase LTK has recently been shown by the present inventors to be an endoplasmic reticulum (ER)-resident kinase that regulates trafficking from the ER to the Golgi (Centonze et al., J Cell Biol 218(8): 2470-2480, 2019). Inhibition or knockdown of LTK was found to result in retardation of ER-to-Golgi trafficking. The present inventors have found that by blocking this pathway, inhibition of LTK is effective in treating cancer associated with protein hypersecretion, in particular multiple myeloma (co-pending application PCT/EP2020/056698).

RNA viruses are known to hijack the secretory pathway: viral proteins are synthesised and glycosylated in the ER, and virions are assembled in compartments distal to the ER such as the ERGIC (ER-Golgi Intermediate Compartment) or the Golgi. Such pathways are known to be used by the influenza virus and coronaviruses (Fung & Liu, Front Microbiol 5: 296, 2014).

Yi et al., Frontiers in microbiology vol. 11 1440, 25 Jun. 2020, discloses the identification of the small molecule compound entrectinib (RAI-13), which was found to inhibit human norovirus, dengue virus and hepatitis C infection.

US2019167710A1 discloses a method comprising administering to a patient having an elevated inflammatory response an inhibitor of anaplastic lymphoma kinase (ALK inhibitors) in an amount effective to treat the elevated inflammatory response.

### Summary

The references to the methods of treatment by therapy in this description are to be interpreted as references to compounds, pharmaceutical compositions and medicaments of the present invention for use in those methods.

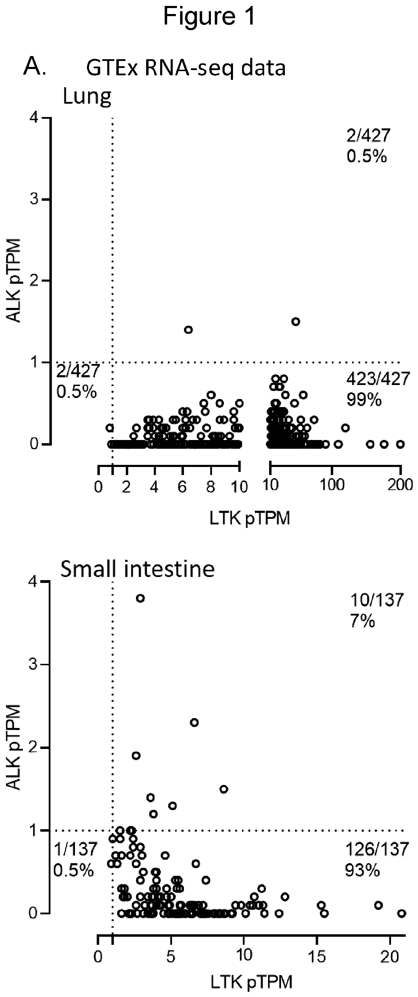

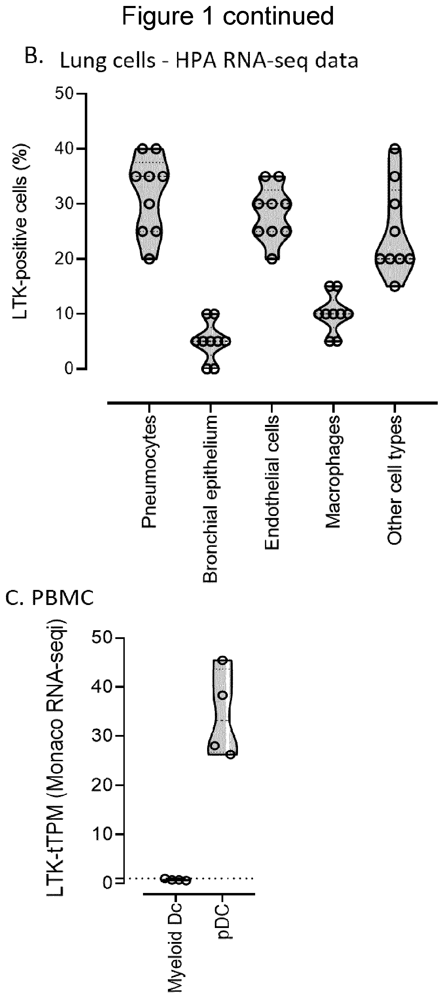

The present disclosure demonstrates that inhibition of LTK blocks assembly and secretion of HPAI and SARS-CoV-2 *in vitro.* It has been found that LTK, but not ALK, is well expressed in lung and GI tissue (see Figure 1). These tissues may be severely affected by influenza virus, SARS-CoV-2 and other RNA viruses. LTK inhibitors can thus be used to treat such infections, and play a key role in control the current and future pandemics. Advantageously the present inventors have found that acyclic ALK inhibitors are also capable of inhibiting LTK (PCT/EP2020/056698). Many acyclic ALK inhibitors have already been licensed for treatment of ALK+ cancers, in particular non-small cell lung cancer (NSCLC), including crizotinib, ceritinib, alectinib, entrectinib and brigatinib. As these drugs are already approved, they can be rapidly repurposed for treatment of viral infections, and they also allow oral administration. The present disclosure thus provides a new broad-spectrum therapeutic option for severe viral infections such as COVID-19, that can be quickly be adopted by the medical community.

In a first aspect, provided herein is a leukocyte tyrosine kinase (LTK) inhibitor for use in the treatment of influenza in a human subject,
wherein the LTK inhibitor is selected from ceritinib, brigatinib, ensartinib, alectinib, crizotinib, entrectinib, belizatinib, CEP-28122, CEP-37440 or ALK-IN-1,
and wherein the influenza is caused by a Type A influenza virus of subtype H1N1, H3N2, H5Nx, H7N1 or H7N9, or a Type B influenza virus.

Also provided herein is an *in vitro* or *ex vivo* method of inhibiting assembly and/or secretion of influenza virus, comprising contacting cells infected with influenza virus or at risk of infection by influenza virus with a leukocyte tyrosine kinase (LTK) inhibitor, wherein the LTK inhibitor is selected from ceritinib, brigatinib, ensartinib, alectinib, crizotinib, entrectinib, belizatinib, CEP-28122, CEP-37440 or ALK-IN-1.

Thus, the contacting may be by contacting isolated cells or tissues *in vitro* or *ex vivo.*

Further provided herein is a leukocyte tyrosine kinase (LTK) inhibitor for use in inhibiting the assembly and/or secretion of influenza virus in a human subject thereby to treat or prevent infection of said subject by the virus,
wherein the LTK inhibitor is selected from ceritinib, brigatinib, ensartinib, alectinib, crizotinib, entrectinib, belizatinib, CEP-28122, CEP-37440 or ALK-IN-1,
and wherein the influenza is caused by a Type A influenza virus of subtype H1N1, H3N2, H5Nx, H7N1 or H7N9, or a Type B influenza virus.

### Detailed Description

Research into treatments for viral infections has traditionally focussed on antiviral therapies that directly target viruses. For instance, as detailed above, common targets for antivirals include viral RNA-dependent RNA polymerases, and in the case of influenza, neuraminidase. In the case of COVID-19 monoclonal neutralising antibodies have also been developed to block viral entry into host cells (Abbasi, JAMA 324(2):128, 2020). The present inventors have adopted the rarer approach of treating viral infections by targeting host cell mechanisms that play a role in viral replication, specifically LTK. Surprisingly, inhibition of LTK has been found to be highly effective in inhibiting assembly/secretion of influenza and SARS-CoV-2, as demonstrated in the examples below.

Provided herein is thus an LTK inhibitor for use in treating a viral infection in a subject, wherein the viral infection is caused by influenza, which is an RNA virus. The term "RNA virus", in line with the standard definition in the art, is used to refer to any virus with an RNA genome (that is to say, a virus with genetic material composed of RNA).

A viral infection is, in line with the standard meaning of the term, an infection caused by a virus. That is to say, the present invention provides a new therapy for infections caused by viruses (specifically infections caused by influenza viruses). As discussed further below, RNA viruses include retroviruses and riboviruses (i.e. RNA viruses that are not retroviruses).

Influenza, the viral infection treated according to the uses and methods herein, is an enveloped RNA virus.

As is well known in the art, all virus particles (or virions) comprise a protein capsid encasing the viral genetic material. The capsid consists of capsid proteins, also known as viral coat proteins. An enveloped virus comprises an additional external layer known as the viral envelope. The viral envelope is a membranous layer derived from the membrane of the host cell in which the virion was formed. The envelope is formed when the virus particle buds off from the host cell, and so comprises host cell membrane and membrane proteins, in addition to viral glycoproteins which recognise host cell surface receptors. Upon binding of the enveloped virus to a new host cell, the envelope fuses with the host cell membrane, causing the centre of the virion (i.e. the capsid and genetic material) to enter the cell.

The majority of RNA viruses are enveloped. Medically-significant enveloped RNA virus families include *Coronaviridae, Orthomyxoviridae, Flaviviridae, Togaviridae, Paramyxoviridae, Rhabdoviridae* and *Filoviridae.* Influenza belongs to the family *Orthomyxoviridae.*

Double-stranded RNA viruses constitute Baltimore classification Group III and have a genome consisting of double-stranded RNA (i.e. complementary positive and negative RNA strands). *Rotavirus* is a medically-significant genus of double-stranded RNA virus, that is the most common cause of gastroenteritis in infants and young children.

Influenza is a single-stranded RNA virus (ssRNA virus, i.e. having a genome consisting of a single RNA strand). More particularly, it is an enveloped ssRNA virus.

A +ssRNA virus has a genome consisting of a single strand of positive-sense RNA. The virus's RNA genome can thus act as messenger RNA (mRNA) in a host cell, and be directly translated into viral proteins.

Positive-sense single-stranded RNA riboviruses constitute Baltimore classification Group IV, and include the family *Coronaviridae* and the family *Flaviviridae.*

Diagnosis of viral diseases can be made by a trained physician, using suitable diagnostic procedures, e.g. physical examination, RT-PCR performed on blood samples, etc. In practice, clinicians may prefer not to intervene in mild cases of disease, and administer treatment only in the case of more serious illness or to at-risk individuals. Such treatment decisions lie within the routine duties of the skilled clinician.

Other positive-sense single-stranded RNA viruses include retroviruses. As is well known to the skilled person, retroviruses are characterised by their use of the enzyme reverse transcriptase to reverse transcribe their RNA genomes into DNA, which is then integrated into the host genome using the integrase enzyme. RNA retroviruses constitute Baltimore classification Group VI.

Negative-sense single-stranded RNA viruses (-ssRNA viruses) constitute another group of single-stranded RNA viruses that cause viral infection, in particular enveloped negative-sense single-stranded RNA viruses. A -ssRNA virus has a genome consisting of a single strand of negative-sense RNA. During infection by a -ssRNA virus, the negative-sense RNA strand acts as template for transcription of mRNA by the viral RNA-dependent RNA polymerase, and during viral replication the same enzyme generates a positive-sense RNA antigenome that is used as template for the synthesis of the negative-sense RNA genome. Negative-sense single-stranded RNA viruses constitute Baltimore classification Group V.

In accordance with the invention, the negative-sense single-stranded RNA virus that causes the viral infection to be treated is an influenza virus. That is to say, an LTK inhibitor may be used to treat influenza in a subject.

As is known to the skilled person, there are four genera of influenza virus: influenza A, influenza B, influenza C and influenza D (also known as Types A-D). Types A-C are known to infect humans. It is unclear whether Type D influenza is capable of infecting humans.

Seasonal flu epidemics are caused by Type A and B influenza viruses. According to the present invention, an LTK inhibitor is used to treat influenza caused by a Type A influenza virus or a Type B influenza virus.

Type B influenza is capable of infecting only humans and seals. Since influenza is a segmented virus (all its genes are encoded on separate viral RNA segments), influenza strains are capable of reassortment, in which two strains mix and exchange vRNA segments, giving rise to a new viral strain. Due to its limited host range, Type B influenza is not capable of antigenic shift (in which two strains of influenza from different host organisms undergo reassortment, yielding a new subtype with a significantly different phenotype). Influenza B is not therefore capable of causing human pandemics (or is not believed to be so capable).

Type A influenza, on the other hand, has a wide host range, being capable of infecting not only humans but also many bird species (both wild and domestic) and several other mammalian species including dogs, horses and pigs. Type A influenza therefore has high potential for antigenic shift, and all known influenza pandemics have been caused by Type A influenza, including the 1918 Spanish flu. A particularly important embodiment is therefore the use of an LTK inhibitor in the treatment of influenza caused by a Type A influenza virus.

Influenza A viruses are classified into subtypes based on their haemagglutinin (H) and neuraminidase (N) serotypes. An LTK inhibitor may be used to treat influenza caused by any Type A influenza virus of any subtype, i.e. having any neuraminidase serotype and any haemagglutinin serotype. Examples of influenza A subtypes include e.g. H1N1 (i.e. having haemagglutinin serotype 1 and neuraminidase serotype 1), the subtype responsible for both the 1918 Spanish flu pandemic and the mild 2009 swine flu pandemic, and H3N2 (i.e. having haemagglutinin serotype 1 and neuraminidase serotype 2), the subtype responsible for the 1968 Hong Kong flu pandemic. As mentioned above, an LTK inhibitor may be used to treat influenza caused by an influenza A subtype, including these.

In recent years and decades certain highly pathogenic strains of avian influenza have developed, i.e. that show high pathogenicity in birds. These strains are referred to as highly pathogenic avian influenza (HPAI). Though no HPAI strain has to-date gained the ability to transmit efficiently between humans, in rare cases of bird-to-human transmission HPAI strains have also shown high pathogenicity in humans. HPAI strains are therefore considered a major risk for future influenza pandemics, should they gain the ability to transmit efficiently between humans. Certain strains of low pathogenic avian influenza (LPAI), i.e. strains that show low pathogenicity in birds, have also been found to show high pathogenicity in humans, and these strains are therefore also considered a major risk for future pandemics.

Accordingly, an LTK inhibitor may be used to treat influenza caused by an avian influenza strain. An LTK inhibitor may be used to treat influenza caused by highly pathogenic avian influenza (HPAI). An LTK inhibitor may be used to treat influenza caused by low pathogenic avian influenza (LPAI).

In recent years, particular strains of avian influenza have caused significant concern as to their potential to cause a pandemic, due to their high pathogenicity in humans. These include H5Nx strains, particularly H5N1, H5N2, H5N6 and H5N8, and H7N9. An LTK inhibitor may be used to treat influenza caused by an H7N1 or H7N9 influenza strain or an H5Nx influenza strain, such as influenza caused by an H5N1, H5N2, H5N6 or H5N8 influenza strain.

According to the present invention, the influenza which is treated is caused by a Type A influenza virus of subtype H1N1, H3N2, H5Nx, H7N1 or H7N9, or a Type B influenza virus.

The methods and uses disclosed herein may have particular utility in treating subjects suffering from seasonal flu. The seasonal flu may be caused by a Type A influenza virus of subtype H1N1, H3N2, H5Nx, H7N1 or H7N9, or a Type B influenza virus. In another embodiment the influenza may be pandemic flu. The pandemic flu may be caused by a Type A influenza virus of subtype H1N1, H3N2, H5Nx, H7N1 or H7N9. In the context of these particular influenza infections the subject may be a human subject.

In certain embodiments, the subject may be a subject who has been hospitalised for the influenza infection. Thus, the subject may be a human subject who is presenting with severe symptoms of influenza, and in particular symptoms which require in-patient hospital care.

An LTK inhibitor, as referred to herein, is a compound which is capable of interacting with LTK (specifically human LTK) and reducing or abrogating its activity. Human LTK has the UniProt accession number P29376. Methods for testing compounds for activity in LTK inhibition are known, and described in Centonze *et al. (supra).* In particular, LTK activation requires autophosphorylation on Tyr672. Inhibition of LTK therefore inhibits Tyr672 autophosphorylation, which may be detected by immunoblot using an anti-phospho-LTK(Y672) antibody. Accordingly, to determine whether a compound has activity in LTK inhibition (i.e. is an LTK inhibitor), the compound of interest may be applied to cells expressing human LTK, the cells lysed and LTK autophosphorylation on Tyr672 analysed.

Many commercial antibodies exist that are suitable for detecting LTK phosphorylated on Tyr672. Human LTK has high homology to human ALK (UniProt accession number Q9UM73). Similarly to LTK, ALK activation requires autophosphorylation on Tyr1278 (Tyr1278 of human ALK is equivalent to Tyr672 of human LTK) and several antibodies that recognise phospho-ALK(Y1278) are commercially available. Many of the commercially available antibodies which recognise phospho-ALK(Y1278) also recognise phospho-LTK(Y672), and can thus be used to determine whether a compound is an LTK inhibitor. An example of such an antibody, which can be used to detect phospho-LTK(Y672), is antibody D59G10, available from Cell Signaling Technology (USA).

Thus the LTK inhibitor for use according to the uses and methods herein may have activity in reducing LTK autophosphorylation of Tyr672, as determined using antibody D59G10 to detect phosphor-LTK(Y672). This determination may be made by any technique known in the art, e.g. immunoblotting (particularly Western blotting).

The LTK inhibitor may completely abrogate LTK autophosphorylation of Tyr672, such that following treatment with the inhibitor phospho-LTK(Y672) is undetectable. Alternatively, the LTK inhibitor may reduce LTK autophosphorylation of Tyr672, but not completely abrogate it. In this case, some phospho-LTK(Y672) is detectable following treatment with the LTK inhibitor, but less phospho-LTK(Y672) is detectable than in a control sample not contacted with an LTK inhibitor. Levels of phospho-LTK(Y672) in a sample may be quantified using any method known in the art. For instance, if phospho-LTK(Y672) detection is performed by immunoblotting, levels of phospho-LTK(Y672) in each sample may be quantified by densitometry. An LTK inhibitor may reduce LTK autophosphorylation of Tyr672 (and thus the level of phospho-LTK(Y672) in a sample) by e.g. at least 50 %, at least 60 %, at least 70 %, at least 80 % or at least 90 %.

As detailed above, many existing ALK inhibitors have been discovered also to have activity in inhibiting LTK. Specifically, acyclic ALK inhibitors (which may alternatively be referred to as linear ALK inhibitors) have been found to have activity in inhibiting LTK, while macrocyclic ALK inhibitors have been found to have no or minimal activity against LTK. In line with standard nomenclature in the art, a macrocyclic molecule is defined herein as a molecule that contains a cyclic framework of at least twelve atoms. As described in Basit et al. (European Journal of Medicinal Chemistry 134: 348-356, 2017), an example of a macrocyclic ALK inhibitor is lorlatinib (Formula I).

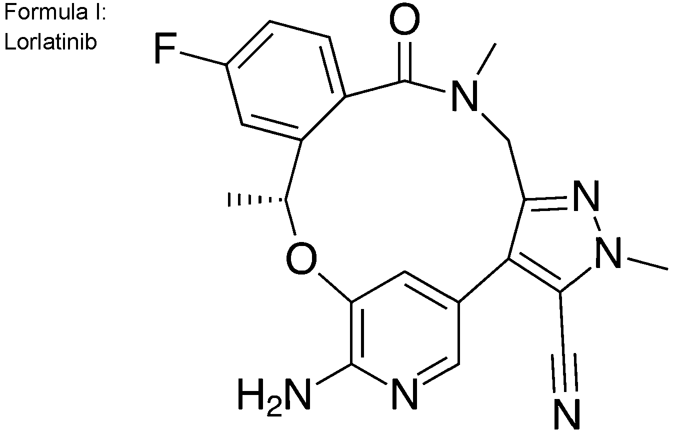

Macrocyclic ALK inhibitors may be referred to as 3^{rd} generation ALK inhibitors. Examples of other macrocyclic ALK inhibitors include repotrectinib and TPX-0131. As noted above, macrocyclic ALK inhibitors have been found to be ineffective in LTK inhibition, and thus are not suitable for use according to the present methods and uses.

As noted above, acyclic ALK inhibitors (which may alternatively be referred to as non-macrocyclic ALK inhibitors) are effective in the inhibition of LTK. The LTK inhibitor for use according to the present uses and methods is an acyclic ALK inhibitor. 1^{st} and 2^{nd} generation ALK inhibitors are acyclic ALK inhibitors. Acyclic ALK inhibitors can in particular be defined as lacking a cyclic framework, as is seen in macrocyclic inhibitors such as lorlatinib. Although an acyclic ALK inhibitor lacks a cyclic framework, as is known to the skilled person, an acyclic ALK inhibitor may nonetheless comprise one or more cyclic functional groups, which may be connected in series.

Acyclic ALK inhibitors which may be used as an LTK inhibitor according to the current invention are ceritinib, brigatinib, ensartinib, alectinib, crizotinib, entrectinib, belizatinib, CEP-28122, CEP-37440 and ALK-IN-1.

In an embodiment, the LTK inhibitors for use herein do not include entrectinib (in other words, in an embodiment the LTK inhibitor is not entrectinib).

In another embodiment, the LTK inhibitor is ceritinib, brigatinib, ensartinib, alectinib, crizotinib, entrectini or belizatinib.

Ceritinib (5-chloro-2-*N*-(5-methyl-4-piperidin-4-yl-2-propan-2-yloxyphenyl)-4-*N*-(2-propan-2-ylsulfonylphenyl)pyrimidine-2,4-diamine) has the trade name Zykadia and the structure shown in Formula II:

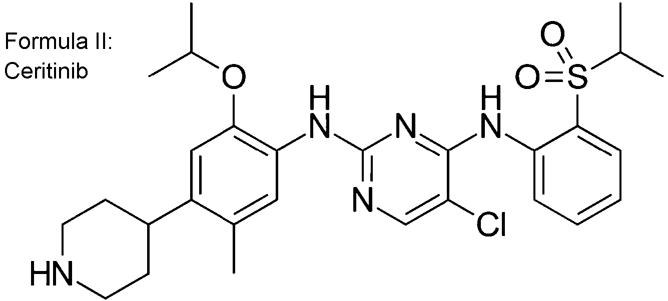

Brigatinib (5-chloro-4-*N*-(2-dimethylphosphorylphenyl)-2-*N*-[2-methoxy-4-[4-(4-methylpiperazin-1-yl)piperidin-1-yl]phenyl]pyrimidine-2,4-diamine) has the trade name Alunbrig and the structure shown in Formula III:

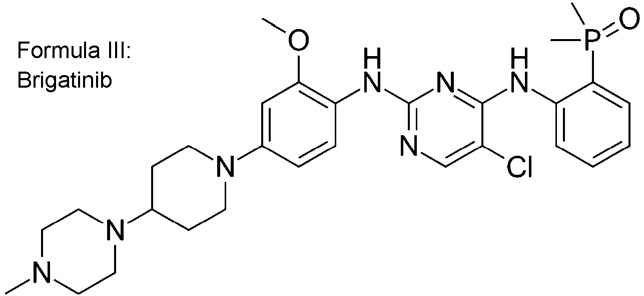

Ensartinib (6-amino-5-[(1R)-1-(2,6-dichloro-3-fluorophenyl)ethoxy]-N-[4-[(3R,5S)-3,5-dimethylpiperazine-1-carbonyl]phenyl]pyridazine-3-carboxamide) has the structure shown in Formula IV:

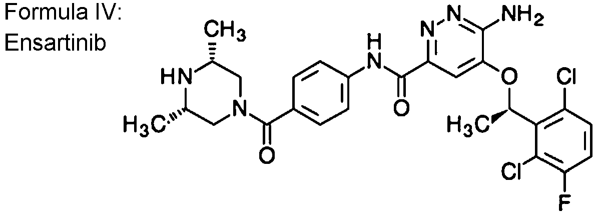

Alectinib (9-ethyl-6,6-dimethyl-8-(4-morpholin-4-ylpiperidin-1-yl)-11-oxo-5H-benzo[b]carbazole-3-carbonitrile) has the trade name Alecensa and the structure shown in Formula V:

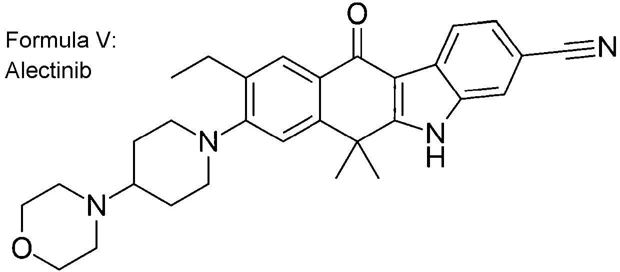

Crizotinib (3-[(1*R*)-1-(2,6-dichloro-3-fluorophenyl)ethoxy]-5-(1-piperidin-4-ylpyrazol-4-yl)pyridin-2-amine) has the trade name Xalkori and the structure shown in Formula VI:

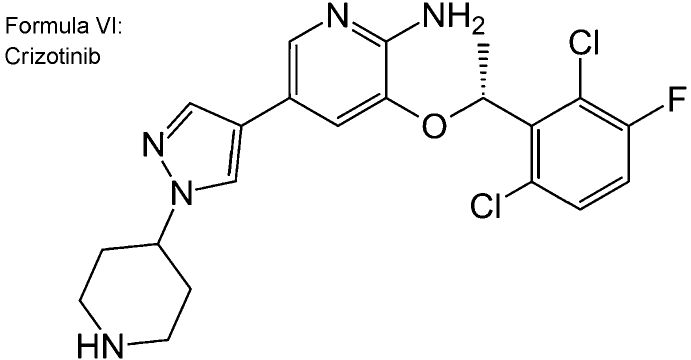

Entrectinib (*N*-[5-[(3,5-difluorophenyl)methyl]-1*H*-indazol-3-yl]-4-(4-methylpiperazin-1-yl)-2-(oxan-4-ylamino)benzamide) has the trade name Rozlytrek and the structure shown in Formula VII:

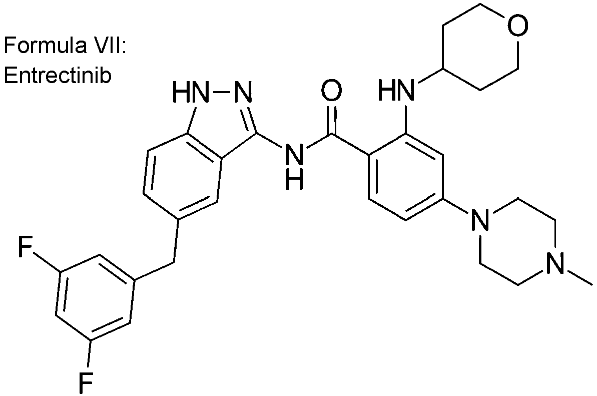

Belizatinib (4-fluoro-N-[6-[[4-(2-hydroxypropan-2-yl)piperidin-1-yl]methyl]-1-[4-(propan-2-ylcarbamoyl)cyclohexyl]benzimidazol-2-yl]benzamide) has the structure shown in Formula VIII:

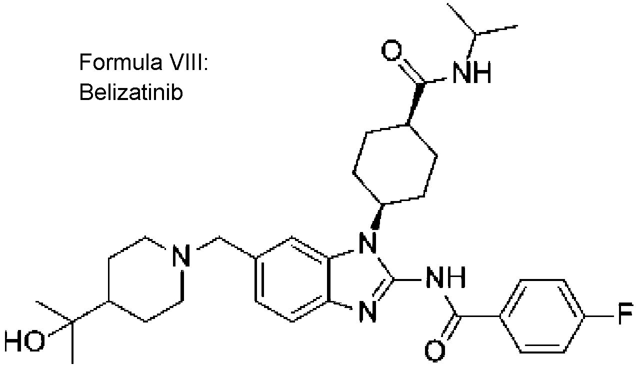

CEP-28122 ((1S,2S,3R,4R)-3-[[5-chloro-2-[[(7S)-6,7,8,9-tetrahydro-1-methoxy-7-(4-morpholinyl)-5H-benzocyclohepten-2-yl]amino]-4-pyrimidinyl]amino]-bicyclo[2.2.1]hept-5-ene-2-carboxamide) has the structure shown in Formula IX:

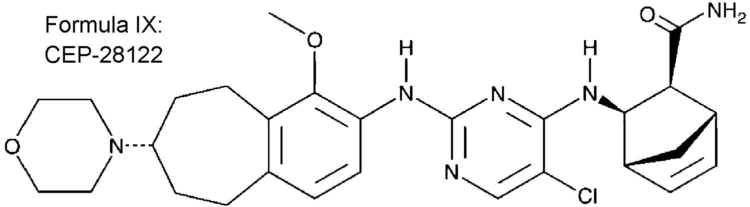

CEP-37440 (2-[[5-chloro-2-[[(6S)-6,7,8,9-tetrahydro-6-[4-(2-hydroxyethyl)-1-piperazinyl]-1-methoxy-5H-benzocyclohepten-2-yl]amino]-4-pyrimidinyl]amino]-N-methyl-benzamide) has the structure shown in Formula X:

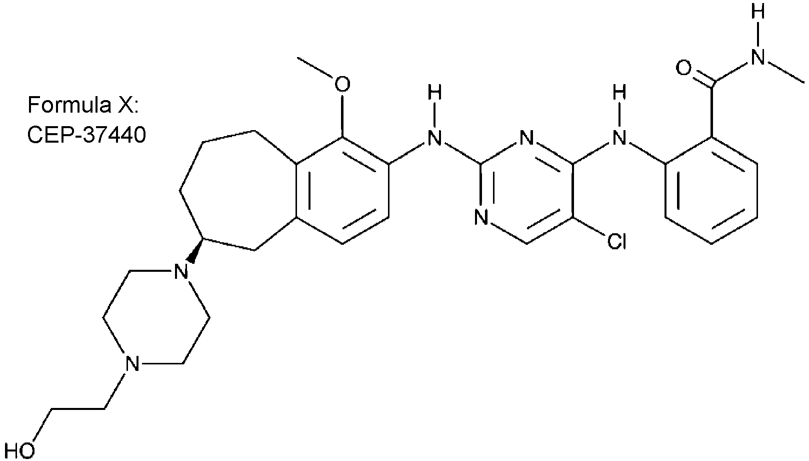

ALK-IN-1 5-chloro-N2-[4-[4-(dimethylamino)-1-piperidinyl]-2-methoxyphenyl]-N4-[2-(dimethylphosphinyl)phenyl]-2,4-pyrimidinediamine) has the structure shown in Formula XI: 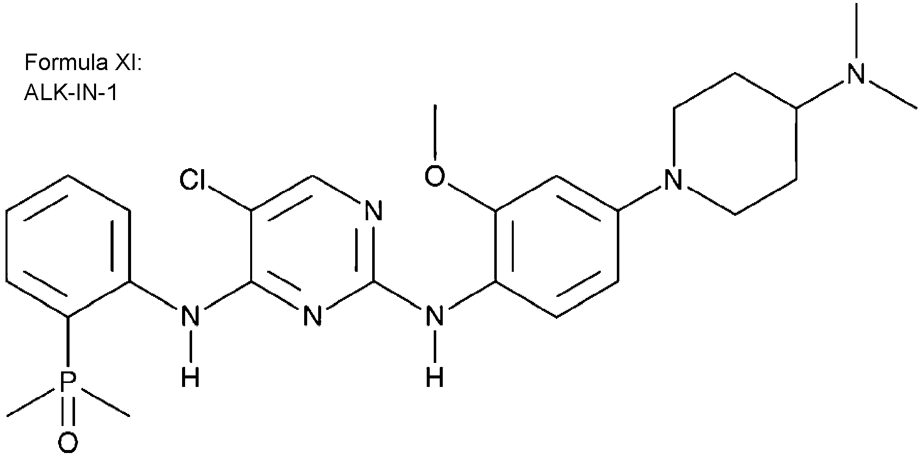 The general structure of an LTK inhibitor is shown in Formula XII: 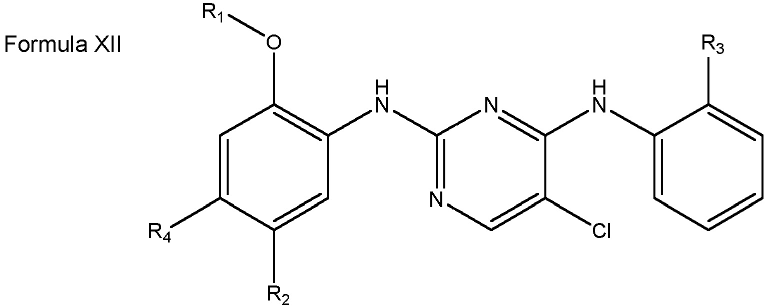 Wherein:
R₁ is CH₃ or iPr;
R₂ is H or CH₃;
R₃ is 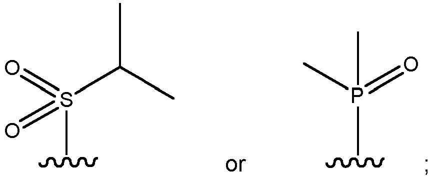 and
R₄ is

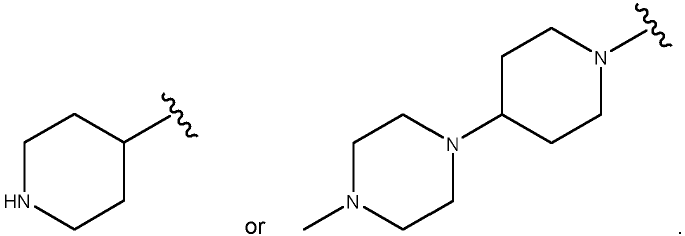

Pharmaceutically acceptable salts or esters, or other pro-drugs of the LTK inhibitors described herein may be used, in accordance with principles well known in the pharmaceutical arts. The identified LTK inhibitors referred to herein by name thus include salts and esters, and pro-drugs more generally, particularly salts and esters thereof. Such pharmaceutically acceptable salts include acid and base addition salts, e.g. with organic or inorganic acids or bases.

In a particular embodiment the LTK inhibitor is ceritinib, brigatinib, ensartinib, alectinib, crizotinib or entrectinib. For treatment of influenza, ceritinib may in particular be used. Alternatively, for treatment of influenza, entrectinib may be used.

The LTK inhibitor is administered to the subject at a pharmaceutically-effective dosage (i.e. a dosage that is sufficient to have an effect on the viral infection being treated).

The existing FDA-approved dosing regimen for ceritinib is for treatment of non-small cell lung cancer (NSCLC), and is a once daily 750 mg oral dose. Ceritinib may be used (i.e. administered to a subject to treat a viral infection caused by an RNA virus) at a dosage of up to 750 mg/day. The maximum ceritinib dose may therefore be e.g. 750, 700, 650, 600, 550, 500, 450, 400, 350, 300, 250, 200, 150 or 100 mg/day. A minimum pharmaceutically-effective dosage of ceritinib to be used may be e.g. 50, 100, 150, 200, 250 or 300 mg/day.

The existing FDA-approved dosing regimen for brigatinib is for treatment of NSCLC, and is a once daily 90 mg oral dose for the first 7 days. If the 90 mg/day dose is tolerated, the dosage is increased to 180 mg/day. Brigatinib may be used (i.e. administered to a subject to treat a viral infection caused by an RNA virus) at an initial dosage of up to 90 mg/day for 7 days, and if tolerated the dosage may be increased to up to 180 mg/day. The maximum initial brigatinib dosage may be 90, 80, 70, 60, 50, 40 or 30 mg/day. The maximum subsequent brigatinib dosage (if the initial dosage is tolerated) may be 180, 160, 140, 120, 100, 80, 60 or 40 mg/day. A minimum pharmaceutically-effective initial dosage of brigatinib to be used may be e.g. 20, 30, 40, 50 or 60 mg/day. A minimum pharmaceutically-effective subsequent dosage of brigatinib (to be used of the initial dosage is tolerated) may be 30, 40, 50, 60, 70, 80 or 90 mg/day.

Ensartinib is currently in phase III clinical trials for treatment of NSCLC. It is being trialled at a dosage of 225 mg/day, orally. Ensartinib may thus be used (i.e. administered to a subject to treat a viral infection caused by an RNA virus) at a dosage of up to 225 mg/day. The maximum ensartinib dose may therefore be 225, 210, 200, 190, 180, 170, 160, 150, 140, 130, 120, 110 or 100 mg/day. A minimum pharmaceutically-effective dosage of ensartinib to be used may be e.g. 80, 90, 100, 110, 120, 130, 140 or 150 mg/day.

The existing FDA-approved dosing regimen for alectinib is for treatment of NSCLC, and is a twice daily oral dose of 600 mg. Alectinib may thus be used (i.e. administered to a subject to treat a viral infection caused by an RNA virus) at a dosage of up to 600 mg twice daily. The maximum alectinib dose may thus be 600, 550, 500, 450, 400, 350 or 300 mg twice daily. A minimum pharmaceutically-effective dosage of alectinib to be used may be e.g. 100, 150, 200, 250, 300, 350 or 400 mg twice daily. Alternatively, dosing may be once daily, or different doses may be administered at different times of the day, as determined by a physician.

The existing FDA-approved dosing regimen for crizotinib is for treatment of NSCLC and is a twice daily oral dose of 250 mg. Crizotinib may thus be used (i.e. administered to a subject to treat a viral infection caused by an RNA virus) at a dosage of up to 250 mg twice daily. The maximum crizotinib dose may thus be 250, 225, 200, 175, 150, 125 or 100 mg twice daily. A minimum pharmaceutically-effective dosage of crizotinib to be used may be e.g. 80, 100, 120, 140 or 150 mg twice daily. Alternatively, dosing may be once daily, or different doses may be administered at different times of the day, as determined by a physician.

The existing FDA-approved dosing regimen for entrectinib is for treatment of NSCLC and other solid tumours, and is a once daily oral dose of 600 mg. Entrectinib may thus be used (i.e. administered to a subject to treat a viral infection caused by an RNA virus) at a dosage of up to 600 mg/day. The maximum entrectinib dose may thus be 600, 550, 500, 450, 400, 350 or 300 mg/day. A minimum pharmaceutically-effective dosage of entrectinib to be used may be e.g. 100, 150, 200, 250, 300, 350 or 400 mg/day.

For each drug, suitable dosing schedules may be selected by the skilled physician, e.g. based on clinical studies or patient condition or response to the drug.

The subject treated is generally and most particularly a human. However, other animals, particularly other mammals, may also be treated, including livestock, domestic or sports animals. For instance, the subject may be a farm animal such as a cow, horse, sheep, pig or goat, or a pet animal such as a cat or dog, or a racing animal such as a horse. For example the subject may be a cat infected with feline leukemia virus (FeLV) the most common fatal pathogen affecting cats worldwide. FeLV is an enveloped, positive-sense, single-stranded RNA retrovirus. In another example, the subject may be a dog infected with canine coronavirus (CCoV), an enveloped, positive-sense, single-stranded RNA virus that can cause severe diarrhoea, vomiting, and anorexia, or canine respiratory coronavirus (CRCoV) that may cause respiratory disease. In another example the subject may be a cow or calf infected with bovine coronavirus (BCV-2 or BCoV-3) that causes calf enteritis/pneumonia entailing profuse diarrhoea, dehydration, depression, reduced weight gain and anorexia, pneumonia and a serous to purulent nasal discharge. In another example the subject may be a horse infected with equine coronavirus (ECoV) an emerging virus that can cause fever, depression, anorexia, colic or diarrhoea, disruption of the gastrointestinal barrier and severe systemic infections in adult horses.

The subject is an individual who has been diagnosed with a viral infection caused by an RNA virus according to the present invention, who may therefore be treated with an LTK inhibitor according to the present uses and methods. The diagnosis may be made by any suitable method known to the skilled person, e.g. RT-PCR performed on a swab, an alternative nucleic acid amplification technique (e.g. LAMP), antigen detection by e.g. ELISA, lateral flow assay, or based on clinical presentation.

The LTK inhibitor may be administered to the subject by any suitable route. Existing ALK inhibitors are currently formulated for oral administration, but alternative administration routes may be utilised as desired. In particular the LTK inhibitor may be formulated for administration to the subject intravenously or as an aerosol. In particular, existing acyclic ALK inhibitors may be reformulated for administration to the subject intravenously or as an aerosol.

The LTK inhibitor is particularly administered in the form of a pharmaceutical composition. Suitable pharmaceutical compositions may include liquid solutions, suspensions or syrups, and solid compositions such as powders, granules, tablets or capsules. In an embodiment, the LTK inhibitor is provided as a liquid solution or suspension for intravenous or aerosol administration. In another embodiment the LTK inhibitor is provided for oral administration.

Pharmaceutically-acceptable diluents, carriers and excipients for use in such pharmaceutical compositions are well known in the art. For instance, suitable excipients include lactose, sodium starch glycolate, maize starch or derivatives thereof, stearic acid or salts thereof, vegetable oils, waxes, fats and polyols. Suitable carriers or diluents include carboxymethylcellulose (CMC), methylcellulose, microcrystalline cellulose, hydroxypropylcellulose, hydroxypropylmethylcellulose (HPMC), dextrose, trehalose, liposomes, polyvinyl alcohol, pharmaceutical grade starch, mannitol, lactose, magnesium stearate, sodium saccharin, talcum, cellulose, glucose, sucrose (and other sugars), magnesium carbonate, silica, gelatin, oil, alcohol, detergents and emulsifiers such as polysorbates. Stabilising agents, wetting agents, emulsifiers, sweeteners etc. may also be used.

Liquid pharmaceutical compositions, whether they be solutions, suspensions or other like form, may include one or more of the following: sterile diluents such as water for injection, saline solution (preferably physiological), Ringer's solution, fixed oils such as synthetic mono- or diglycerides which may serve as a solvent or suspending medium, polyethylene glycols, glycerin, propylene glycol or other solvents; antibacterial agents such as benzyl alcohol or methyl paraben; antioxidants such as ascorbic acid or sodium bisulfite; chelating agents such as EDTA; buffers such as acetates, citrates or phosphates and agents for the adjustment of tonicity such as sodium chloride or dextrose. A parenteral preparation (e.g. for intravenous administration) can be enclosed in ampoules, disposable syringes or multiple dose vials made of glass or plastic. A solution for aerosol administration may be provided in a nebuliser or inhaler. A pharmaceutical composition is typically sterile.

The existing acyclic ALK inhibitors which may be used according to the present uses and methods may be formulated with the excipients and suchlike for which their use has previously been approved. Such information can be found on the regulatory approvals for these drugs issued by the FDA or EMA.

The LTK inhibitor may be used as a monotherapeutic agent (i.e. it may be the only drug used to treat the viral infection). More commonly, the LTK inhibitor may be used in combination with one or more additional active agents to treat the viral infection. The additional active agent(s) used to treat the viral infection in combination with the LTK inhibitor may be any agent useful in (or that may be useful in) treatment of the viral infection.

For instance, the LTK inhibitor may be used in combination with an antiviral drug. For instance, in the treatment of influenza, the LTK inhibitor may be used in combination with one or more anti-flu drugs, such as a neuraminidase inhibitor (e.g. oseltamivir, zanamivir or peramivir) or viral RNA polymerase inhibitor (e.g. favipiravir or baloxavir marboxil). In the treatment of a coronavirus infection, the LTK inhibitor may be used with one or more drugs useful in the treatment of such infections, such a viral RNA polymerase inhibitor (e.g. remdesivir). Any other suitable antiviral drug may also be used in combination with an LTK inhibitor.

Alternatively, the LTK inhibitor may be used in combination with another drug that targets host mechanisms associated with the viral infection. For instance, in the case of a viral infection associated with a cytokine storm (e.g. COVID-19), the LTK inhibitor may be used in combination with an immunosuppressant, such as a corticosteroid (e.g. dexamethasone, hydrocortisone or methylprednisolone).

As noted above, the methods and uses herein are predicated at least in part on the effect of LTK inhibitors of inhibiting viral assembly and/or secretion. Thus, in this manner the replication, or more particularly the multiplication or reproduction, of the RNA virus may be inhibited, e.g. prevented, blocked or reduced. In other words the production of virus (or virions or virus particles) in the infected subject may be inhibited. This may be of benefit in the treatment of infected subjects. Thus, as noted above, viewed from another aspect the disclosures herein can be seen to provide methods and uses for inhibiting assembly and/or secretion of an RNA virus in a subject, particularly a human subject. In particular, such methods and uses may be for inhibiting assembly and/or secretion of an RNA virus in a subject, particularly a human subject, thereby to treat or prevent infection of said subject by the virus. Alternatively viewed, the methods and uses may be defined as being for inhibiting production or multiplication of the virus in the subject. In accordance with the present invention, the RNA virus is an influenza virus. More particularly, the influenza virus is a Type A influenza virus of subtype H1N1, H3N2, H5Nx, H7N1 or H7N9, or a Type B influenza virus.

The invention may be further understood by reference to the examples below, and the figures.

### Figures

Figure 1 shows that LTK but not ALK is expressed in lung and GI tissue. A Gene expression data derived from donors to the Genotype-Tissue Expression (GTEx) project (https://www.gtexportal.org/) were analysed for expression of LTK and ALK as accessed in the Human Protein Atlas for corresponding genes. Top: Lung tissue expression of LTK and ALK in 427 donor lungs, a cut-off of transcripts per million (TPM) = 1 is indicated by the dotted line. Bottom: LTK and ALK expression in 137 small intestine samples. B mRNA seq results (Uhlén et al., Science 347: 1260419, 2015) from lung tissue cells were analysed; expression of LTK is shown. C Expression of LTK in flow-sorted subsets from healthy donor PBMCs in the Monaco dataset (Monaco et al., Cell Reports 26: 1627-1640.e7, 2019).

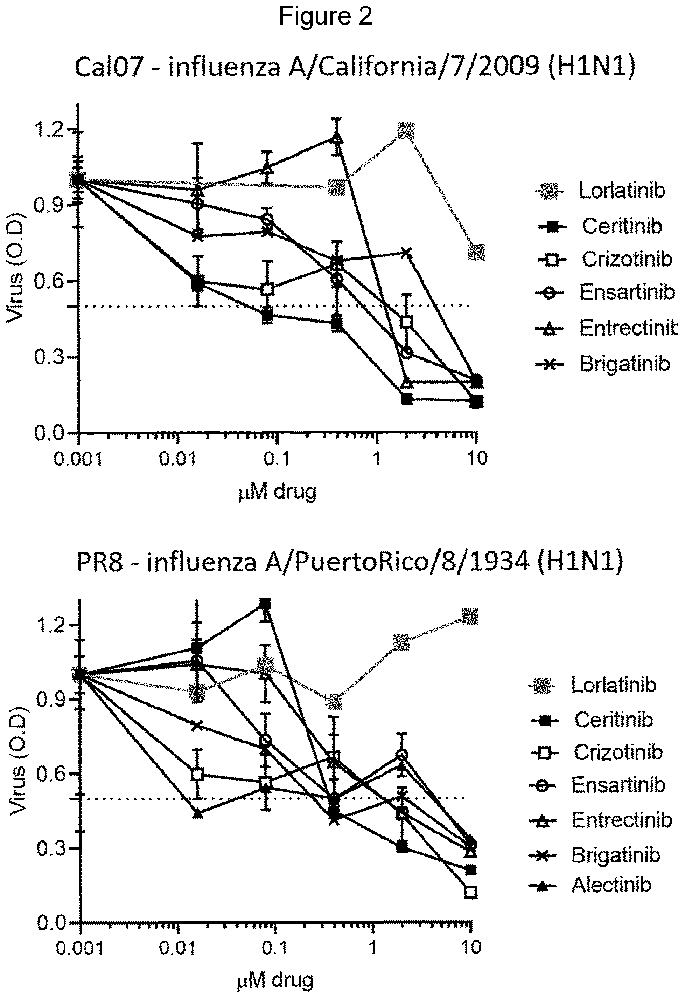

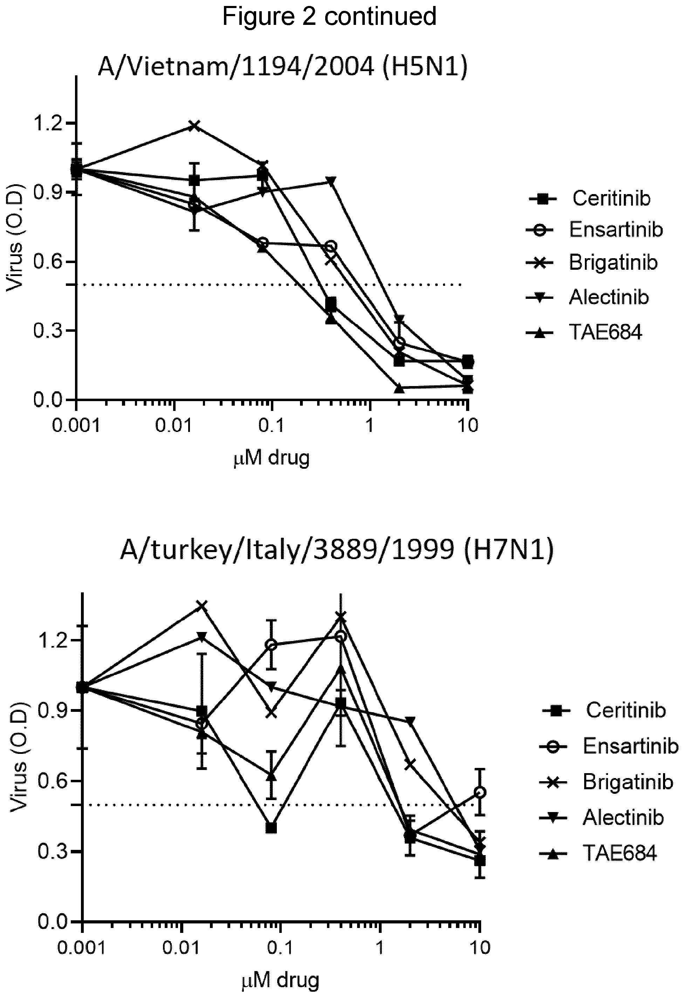

Figure 2 shows that LTK inhibitors inhibit influenza virus assembly/secretion. 5-fold dilutions of the different indicated compounds were added to wells in duplicate. Influenza virus A/California/7/2009 (H1N1) (Cal07), influenza A/PuertoRico/8/1934 (H1N1) (PR8), influenza A/Vietnam/1194/2004 (H5N1), or influenza A/turkey/ltaly/3889/1999 (H7N1) were next added to the titrated drugs, and the mixture added to Madin-Darby canine kidney (MDCK) cells after a 1 h incubation. Following a 50 h incubation, cells were fixed and virus quantified by ELISA against the influenza nucleoprotein.

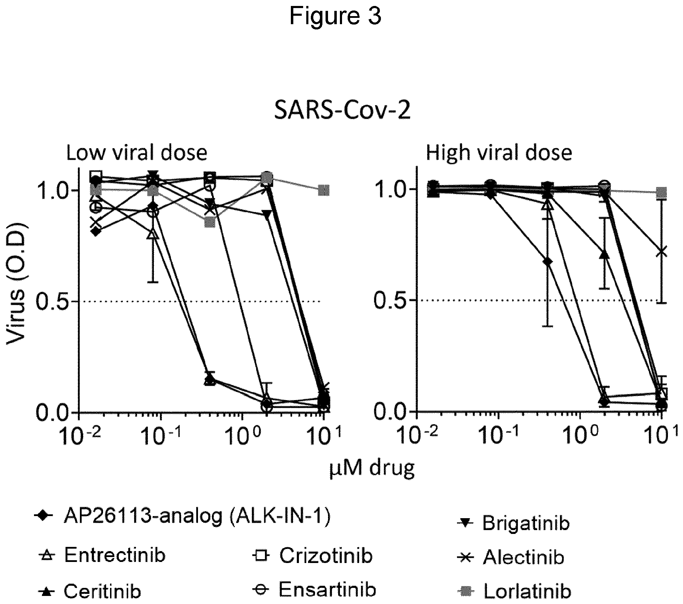

Figure 3 shows that LTK inhibitors reduce SARS-CoV-2 virus assembly/secretion. 5-fold dilutions of the different indicated compounds were added to the wells in duplicate. SARS-CoV-2 virus in low or high titre was added to the titrated drugs, and the mixture added to cultured Vero E6 cells after a 1 h incubation. Following a 50 h incubation, cells were fixed and virus quantified by ELISA against the SARS-CoV-2 nucleocapsid.

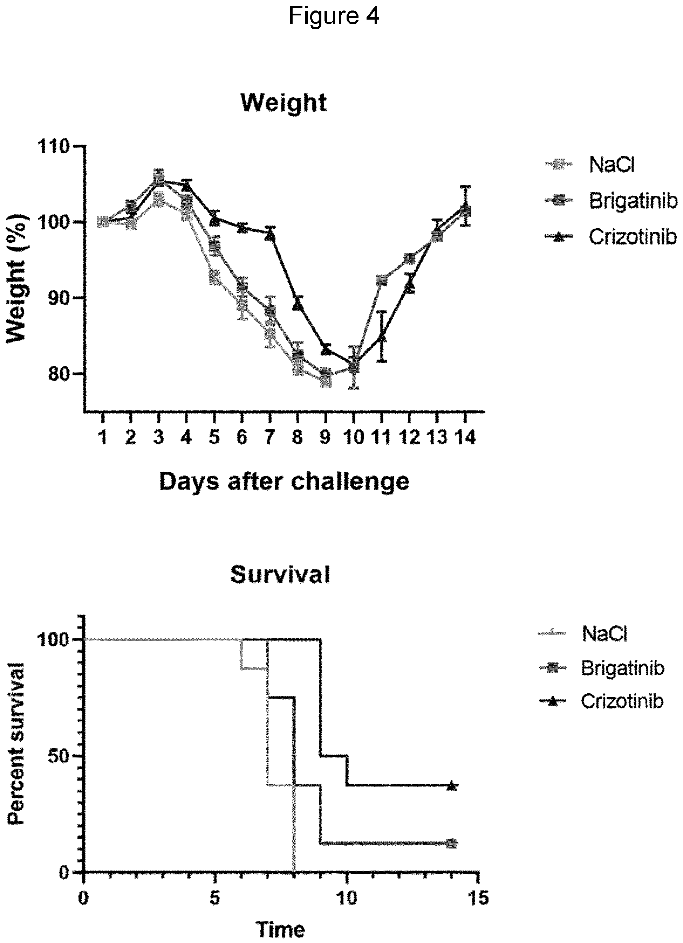

Figure 4 shows that LTK inhibitors provide protection against influenza H1N1. BALB/c mice were infected with 5xLD50 of influenza A/PuertoRico/1934 (H1N1) on day 0. From day 1 until day 7, mice were orally administered the indicated drugs (25mg/kg) once daily. Top: Weight loss in mice after receiving a viral challenge. Bottom: Survival of the mice receiving NaCl, Brigatinib or Crizotinib.

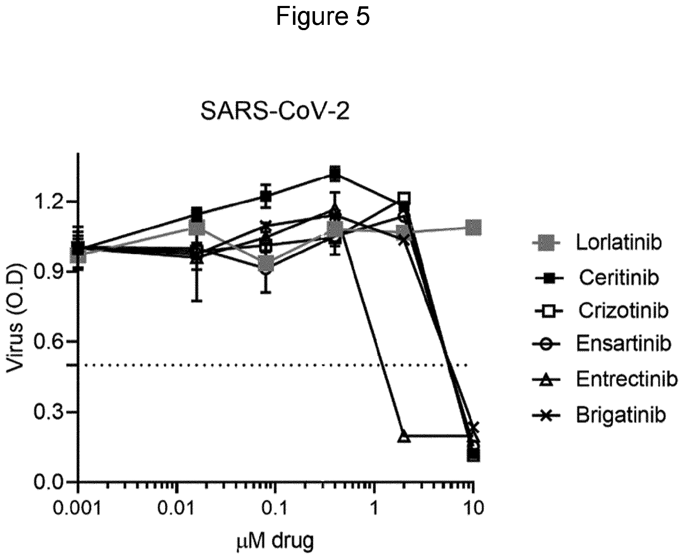

Figure 5 shows that LTK inhibitors inhibit SARS-CoV-2 virus assembly and secretion. Titrated amounts of the indicated drugs were mixed with SARS-CoV-2 virus and incubated for 1 h. Next, the mixture was added to cultured Vero E6 cells. Following a 50 h incubation, cells were fixed and virus quantified by ELISA against the SARS-CoV-2 nucleocapsid.

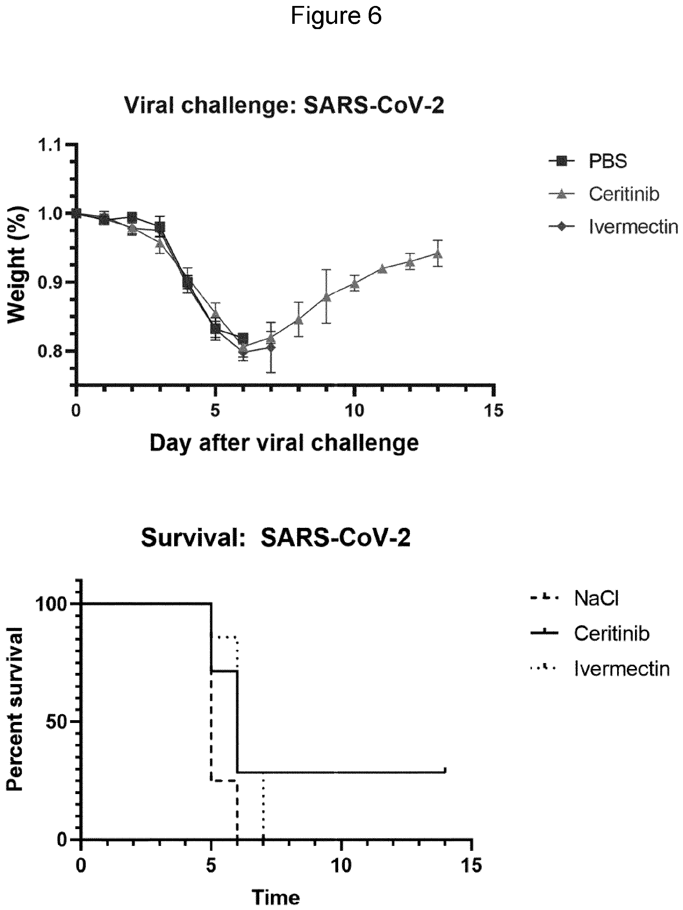

Figure 6 shows that LTK inhibitors provides protection against SARS-CoV-2. F1 mice of BALB/c/C57BL6 mice were infected with 5xLD50 of SARS-CoV-2 on day 0. From day 1 until day 7, mice were orally adminstered the indicated drugs (25mg/kg) once daily. Top: Weight loss in mice after receiving a viral challenge. Bottom: Survival of the mice receiving NaCl, Ceritinib or Ivermectin.

### Examples

Examples relating to the treatment of coronavirus infection and examples relating to the use of Lorlatinib or TAE684 are reference examples.

### Methods

Madin-Darby canine kidney (MDCK) cells or Vero E6 cells were plated out into 96-well cell culture plates at 1x10⁴ cells/well. The next day 100xTCID (tissue culture infective dose) of virus (influenza A/California/07/2009 (H1N1), influenza A/PR/8/1934 (H1N1), influenza A/Vietnam/1194/2004 (H5N1), influenza A/turkey/ltaly/3889/1999 (H7N1), or SARS-CoV-2) were mixed with 5-fold dilutions of titrated drugs. Following a 1 h incubation 37°C in a 5 % CO₂ humidified atmosphere, the mixture was added to the plated cells.

Plates were incubated for 50 h at 37°C in a 5 % CO₂ humidified atmosphere. Next, monolayers were washed with PBS, and fixed in cold 80 % acetone for 10 min. Influenza virus was detected by ELISA using biotinylated mAb against influenza nucleoprotein (HB75, ATCC) and streptavidin-alkaline phosphatase (GE Healthcare). Plates were developed using phosphatase substrate (P4744-10G, Sigma-Aldrich) dissolved in substrate buffer, and read with a Tecan reader using the Magellan v5.03 program.

The SARS-CoV-2 virus Human 2019-nCoV strain 2019-nCoV/ltaly-InMl1 (008V-03893) from the European Virus Archive (EVA) was detected in Vero E6 cell cultures by ELISA using a mAb against SARS-CoV-2 nucleocapsid (40143-R004, SinoBiological) and HRP-conjugated goat anti-rabbit IgG-Fc mAb (SSA003, SinoBiological). Plates were developed using TMB substrate buffer (sc-286967, Santa Cruz), and read with the Tecan reader.

### Results

### LTK but not ALK is Expressed by Human Viral Target Tissues

The expression of LTK and ALK in relevant tissues was tested by analysing data depositories. Both influenza and SARS-CoV-2 target respiratory epithelium and alveolar cells in the lungs. The SARS-CoV-2 virus also infects gastrointestinal tissue via the viral entry receptor ACE2. LTK but not ALK was abundantly expressed in lung tissue in 423/427 donors. 2 donors were double positive, and 2 donors expressed neither LTK nor ALK. A similar dominance was found in the small intestine where 126/137 samples were LTK+ALK-(Figure 1). Most cells from the lung were LTK positive and the plasmacytoid dendritic cells (that endocytose viral particles as part of the first line of defence) were high expressers of LTK.

### LTK Inhibitors Block Viral Assembly of Influenza and SARS-CoV-2 Viruses

The effect of LTK inhibitors on virus assembly/secretion was tested by adding influenza virus to MDCK **(****Figure 2****)** or SARS-CoV-2 to VERO E6 cells **(****Figure 3** **and** **Figure 5****).** This assay measures the virus that has replicated in the cells during the 50 hour incubation. If the drug has a potential to prevent viral growth, this is seen as a lower viral OD (y-axis) in the figures. Of the influenza viruses, both H1N1 viruses and the H5N1 virus were highly sensitive to the LTK inhibitors. The results in respect of the H7N1 virus were less consistent, but also showed an effect on virus assembly/secretion by the LTK inhibitors. Previous work has demonstrated that the cyclic ALK-inhibitor lorlatinib differs from other ALK-inhibitors in that it has low activity towards LTK. Lorlatinib therefore served as a negative control (virus is not prevented from multiplying in cells, and so the viral titres remain high for all doses tested). The other inhibitors had IC50s below or within their therapeutic windows (1-4µM, crizotinib entrectinib, ensartinib, entrectinib, brigatinib, alectinib; and 0.1-1µM ceritinib, against the H1N1 and H5N1 strains). TAE684 was a precursor with harmful metabolites that was modified by Novartis to generate ceritinib.

Inhibition of SARS-CoV-2 was tested in cultures of VERO E6 cells. The LTK inhibitors, but not lorlatinib, inhibited SARS-CoV-2 within the 50 h incubation time **(****Figure 3** **and** **Figure 5****).**

### LTK Inhibitors Provide Protection Against Influenza H1N1 and SARS-CoV-2 Viruses

The protective capacity of LTK inhibitors was tested by infecting BALB/c mice with 5xLD50 of influenza A/PuertoRico/1934 (H1N1) (Figure 4) or by infecting the F1 mice of BALB/c/C57BL6 mice with 5xLD50 of SARS-CoV-2 (Figure 6). Weight loss was seen in all mice which confirms that the very high viral dose has indeed had its effect **(****Figure 4** **(top) and** **Figure 6** **(top)).** The survival of mice receiving Crizotinib (38%) and Brigatinib (12.5%) **(****Figure 4** **(bottom))** and Ceritinib (29%) **(****Figure 6** **(bottom))** confirms that these drugs have a protective efficacy even against a dose far higher than what can be considered relevant in the context of exposure to the virus in a natural environment.

## Claims

1. A leukocyte tyrosine kinase (LTK) inhibitor for use in the treatment of influenza in a human subject,
wherein the LTK inhibitor is selected from ceritinib, brigatinib, ensartinib, alectinib, crizotinib, entrectinib, belizatinib, CEP-28122, CEP-37440 or ALK-IN-1,
and wherein the influenza is caused by a Type A influenza virus of subtype H1N1, H3N2, H5Nx, H7N1 or H7N9, or a Type B influenza virus.

2. The LTK inhibitor for use according to claim 1, wherein the influenza virus is an H5Nx or H7N9 influenza virus.

3. The LTK inhibitor for use according to claim 1, wherein the influenza virus is Type A influenza virus of subtype H1N1 or H3N2, or Type B influenza virus.

4. The LTK inhibitor for use according to any one of claims 1 to 3, wherein the LTK inhibitor inhibits assembly and/or secretion of the virus.

5. The LTK inhibitor for use according to any one of claims 1 to 4, wherein the inhibitor is formulated for administration to the subject intravenously or as an aerosol.

6. The LTK inhibitor for use according to any one of claims 1 to 5, wherein the LTK inhibitor is administered to the subject at a pharmaceutically-effective dosage, and wherein:
(i) the LTK inhibitor is ceritinib, and is administered to the subject at a dosage of up to 750 mg/day;
(ii) the LTK inhibitor is brigatinib, and is administered to the subject at a dosage of up to 90 mg/day for 7 days, and if tolerated is thereafter administered to the subject at a dosage of up to 180 mg/day;
(iii) the LTK inhibitor is ensartinib, and is administered to the subject at a dosage of up to 225 mg/day;
(iv) the LTK inhibitor is alectinib, and is administered to the subject at a dosage of up to 600 mg twice daily; or
(v) the LTK inhibitor is crizotinib, and is administered to the subject at a dosage of up to 250 mg twice daily.

7. The LTK inhibitor for use according to any one of claims 1 to 4, wherein the LTK inhibitor is entrectinib, and is administered to the subject at a dosage of up 600 mg/day.

8. An *in vitro* or *ex vivo* method of inhibiting assembly and/or secretion of influenza virus, comprising contacting cells infected with influenza virus or at risk of infection by influenza virus with a leukocyte tyrosine kinase (LTK) inhibitor, wherein the LTK inhibitor is selected from ceritinib, brigatinib, ensartinib, alectinib, crizotinib, entrectinib, belizatinib, CEP-28122, CEP-37440 or ALK-IN-1.

9. The method of claim 8, wherein the influenza virus is a Type A influenza virus of subtype H1, H3, H5 or H7, or a Type B influenza virus.

10. A leukocyte tyrosine kinase (LTK) inhibitor for use in inhibiting the assembly and/or secretion of influenza virus in a human subject thereby to treat or prevent infection of said subject by the virus,
wherein the LTK inhibitor is selected from ceritinib, brigatinib, ensartinib, alectinib, crizotinib, entrectinib, belizatinib, CEP-28122, CEP-37440 or ALK-IN-1,
and wherein the influenza is caused by a Type A influenza virus of subtype H1N1, H3N2, H5Nx, H7N1 or H7N9, or a Type B influenza virus.

## Patentansprüche

1. Leukozyten-Tyrosinkinase-(LTK)-Inhibitor zur Verwendung bei der Behandlung von Influenza bei menschlichen Patienten,
wobei der LTK-Inhibitor aus Ceritinib, Brigatinib, Ensartinib, Alectinib, Crizotinib, Entrectinib, Belizatinib, CEP-28122, CEP-37440 oder ALK-IN-1 ausgewählt ist,
und wobei die Influenza durch ein Influenzavirus vom Typ A des Subtyps H1N1, H3N2, H5Nx, H7N1 oder H7N9 oder ein Influenzavirus vom Typ B verursacht wird.

2. LTK-Inhibitor zur Verwendung nach Anspruch 1, wobei das Influenzavirus ein H5Nx- oder H7N9-Influenzavirus ist.

3. LTK-Inhibitor zur Verwendung nach Anspruch 1, wobei das Influenzavirus ein Influenzavirus vom Typ A des Subtyps H1N1 oder H3N2 oder ein Influenzavirus vom Typ B ist.

4. LTK-Inhibitor zur Verwendung nach einem der Ansprüche 1 bis 3, wobei der LTK-Inhibitor die Assemblierung und/oder Sekretion des Virus hemmt.

5. LTK-Inhibitor zur Verwendung nach einem der Ansprüche 1 bis 4, wobei der Inhibitor für die intravenöse Verabreichung oder als Aerosol an den Patienten formuliert ist.

6. LTK-Inhibitor zur Verwendung nach einem der Ansprüche 1 bis 5, wobei der LTK-Inhibitor dem Patienten in einer pharmazeutisch wirksamen Dosierung verabreicht wird, und wobei:
(i) der LTK-Inhibitor Ceritinib ist und dem Patienten in einer Dosierung von bis zu 750 mg/Tag verabreicht wird;
(ii) der LTK-Inhibitor Brigatinib ist und dem Patienten 7 Tage lang in einer Dosierung von bis zu 90 mg/Tag verabreicht wird und wenn er vertragen wird, dem Patienten danach in einer Dosierung von bis zu 180 mg/Tag verabreicht wird;
(iii) der LTK-Inhibitor Ensartinib ist und dem Patienten in einer Dosierung von bis zu 225 mg/Tag verabreicht wird;
(iv) der LTK-Inhibitor Alectinib ist und dem Patienten in einer Dosierung von bis zu 600 mg zweimal täglich verabreicht wird; oder
(v) der LTK-Inhibitor Crizotinib ist und dem Patienten in einer Dosierung von bis zu 250 mg zweimal täglich verabreicht wird.

7. LTK-Inhibitor zur Verwendung nach einem der Ansprüche 1 bis 4, wobei der LTK-Inhibitor Entrectinib ist und dem Patienten in einer Dosierung von bis zu 600 mg/Tag verabreicht wird.

8. *In-vitro-* oder *Ex-vivo-Verfahren* zur Hemmung der Assemblierung und/oder Sekretion von Influenzaviren, umfassend das Inkontaktbringen von mit Influenzaviren infizierten oder von einer Infektion mit Influenzaviren bedrohten Zellen mit einem Leukozyten-Tyrosinkinase-(LTK)-Inhibitor, wobei der LTK-Inhibitor aus Ceritinib, Brigatinib, Ensartinib, Alectinib, Crizotinib, Entrectinib, Belizatinib, CEP-28122, CEP-37440 oder ALK-IN-1 ausgewählt ist.

9. Verfahren nach Anspruch 8, wobei das Influenzavirus ein Influenzavirus vom Typ A des Subtyps H1, H3, H5 oder H7 oder ein Influenzavirus vom Typ B ist.

10. Leukozyten-Tyrosinkinase-(LTK)-Inhibitor zur Verwendung bei der Hemmung der Assemblierung und/oder Sekretion des Influenzavirus in einem menschlichen Patienten, um dadurch eine Infektion des Patienten mit dem Virus zu behandeln oder zu verhindern,
wobei der LTK-Inhibitor aus Ceritinib, Brigatinib, Ensartinib, Alectinib, Crizotinib, Entrectinib, Belizatinib, CEP-28122, CEP-37440 oder ALK-IN-1 ausgewählt ist,
und wobei die Influenza durch ein Influenzavirus vom Typ A des Subtyps H1N1, H3N2, H5Nx, H7N1 oder H7N9 oder ein Influenzavirus vom Typ B verursacht wird.

## Revendications

1. Inhibiteur de la tyrosine kinase leucocytaire (LTK) destiné à être utilisé dans le traitement de la grippe chez un sujet humain,
l'inhibiteur de LTK étant sélectionné parmi ceritinib, brigatinib, ensartinib, alectinib, crizotinib, entrectinib, belizatinib, CEP-28122, CEP-37440 ou ALK-IN-1,
et la grippe étant provoquée par un virus grippal de type A du sous-type H1N1, H3N2, H5Nx, H7N1 ou H7N9, ou un virus grippal de type B.

2. Inhibiteur de LTK destiné à être utilisé selon la revendication 1, le virus de la grippe étant un virus de la grippe H5Nx ou H7N9.

3. Inhibiteur de LTK destiné à être utilisé selon la revendication 1, le virus de la grippe étant un virus grippal de type A du sous-type H1N1 ou H3N2, ou un virus grippal de type B.

4. Inhibiteur de LTK destiné à être utilisé selon l'une quelconque des revendications 1 à 3, l'inhibiteur de LTK inhibant l'assemblage et/ou la sécrétion du virus.

5. Inhibiteur de LTK destiné à être utilisé selon l'une quelconque des revendications 1 à 4, l'inhibiteur étant formulé pour une administration au sujet par voie intraveineuse ou sous forme d'aérosol.

6. Inhibiteur de LTK destiné à être utilisé selon l'une quelconque des revendications 1 à 5, l'inhibiteur de LTK étant administré au sujet à une dose pharmaceutiquement efficace, et :
(i) l'inhibiteur de LTK étant ceritinib, et étant administré au sujet à une dose allant jusqu'à 750 mg/jour ;
(ii) l'inhibiteur de LTK est brigatinib, et est administré au sujet à une dose allant jusqu'à 90 mg/jour pendant 7 jours, et si toléré est par la suite administré au sujet à une dose allant jusqu'à 180 mg/jour ;
(iii) l'inhibiteur de LTK est ensartinib, et est administré au sujet à une dose allant jusqu'à 225 mg/jour ;
(iv) l'inhibiteur de LTK est alectinib, et est administré au sujet à une dose allant jusqu'à 600 mg deux fois par jour ; ou
(v) l'inhibiteur de LTK est crizotinib, et est administré au sujet à une dose allant jusqu'à 250 mg deux fois par jour.

7. Inhibiteur de **LTK** destiné à être utilisé selon l'une quelconque des revendications 1 à 4, l'inhibiteur de LTK étant entrectinib, et étant administré au sujet à une dose allant jusqu'à 600 mg/jour.

8. Procédé *in vitro* ou *ex vivo* inhibant l'assemblage et/ou la sécrétion du virus de la grippe, comprenant la mise en contact de cellules infectées par le virus de la grippe ou à risque d'infection par le virus de la grippe avec un inhibiteur de la tyrosine kinase leucocytaire (LTK), dans lequel l'inhibiteur de LTK est sélectionné parmi ceritinib, brigatinib, ensartinib, alectinib, crizotinib, entrectinib, belizatinib, CEP-28122, CEP-37440 ou ALK-IN-1.

9. Procédé selon la revendication 8, dans lequel le virus de la grippe est un virus grippal de type A du sous-type H1, H3, H5 ou H7, ou un virus grippal de type B.

10. Inhibiteur de la tyrosine kinase leucocytaire (LTK) destiné à être utilisé pour inhiber l'assemblage et/ou la sécrétion du virus de la grippe chez un sujet humain afin de traiter ou de prévenir l'infection dudit sujet par le virus,
l'inhibiteur de LTK étant sélectionné parmi ceritinib, brigatinib, ensartinib, alectinib, crizotinib, entrectinib, belizatinib, CEP-28122, CEP-37440 ou ALK-IN-1,
et la grippe étant provoquée par un virus grippal de type A du sous-type H1N1, H3N2, H5Nx, H7N1 ou H7N9, ou un virus grippal de type B.
